# EUROPEAN PATENT APPLICATION

(11) **EP 4 850 723 A1**
(43) Date of publication of application: **09.12.2026**
(21) Application number: 25181078.4
(22) Date of filing: 05.06.2025
(51) Int. Cl.: A61M 5/32

(54) **AN IMPROVED CASSETTE UNIT FOR A DRUG DELIVERY DEVICE**

(71) Applicant: Ypsomed AG, 3401 Burgdorf (CH)
(72) Inventor: Klötzli, Urs, 3414 Oberburg (CH)

(57) **Abstract**

The present invention describes a cassette unit (10) for an injection device, the cassette unit (101) comprising: a sleeve (11) defining a longitudinal axis (L) with a proximal opening (16), a holder (13) integrated coaxially in the sleeve (11) and prepared to accommodate a syringe (30), a cap (12) removably attached to the distal end of the sleeve (11), a proximally open tube (14) coaxially with the longitudinal axis (L), the tube (14) having a wall (14a) defining a hole (14b) wherein the tube (14) is constructed from an elastic material and the wall (14a) forms at least three bores (14c) distributed around the circumference of the wall (14a) and an apparatus (20) to assemble a syringe (30) in a cassette unit (10) for an injection device, the apparatus (20) comprising: a base (25), a ram (27), at least three spreaders (23), each spreader (23) having a mandrel (23a) and steps of a method (100) to assemble a syringe (30) in a cassette unit (10) for an injection device carried out on the apparatus (20).

## Description

### FIELD OF THE INVENTION

The present invention relates to drug delivery devices, in particular to cassette or reservoir units with a needle shield remover that is configured to remove a needle shield from a device and to an apparatus and method for applying a needle shield remover to a medicament delivery device e.g. to a syringe or to a reservoir unit of drug delivery device. Advantageous variants of the invention include an improved apparatus or an improved method of manufacturing a drug delivery device.

### BACKGROUND OF THE INVENTION

A variety of diseases exist that require regular treatment by subcutaneous administration of a medicament, and several drug delivery devices have been developed to support a patient in accurately, safely and controllably delivering an amount of drug in a self-administration process. Delivery devices include drug delivery devices that are removed from the injection site after each medication event or drug delivery process, as well as infusion devices with a cannula or needle that remains in the skin of the patient for a prolonged period of time. By way of example, diabetes may be treated by administration of insulin by the patients themselves with the help of multi-variable-dose insulin injection pens, infusion pumps or autoinjectors. Alternatively, patch injectors, wearable injectors or wearable pumps are patched or adhered to the skin of the patient.

Departing from classical syringes, increasingly complex devices have been designed to support different therapies, to ensure safety and reliability, and to increase ease of use to a point patients can apply the drugs themselves, reducing time-consuming and costly interventions by trained medical staff to a minimum. Examples of drug delivery devices suitable for self-treatment include injection pens, auto-injectors, safety syringes, portable infusion pumps and wearable patch pumps. Despite of the technical complexity, it is an important requirement to keep cost of manufacturing and cost of devices as low as possible.

Common to all devices for subcutaneous drug delivery is a reservoir to store the fluid medicament, and a fluid path or cannula to bring the drug out of the device and into the subcutaneous tissue of a patient. Fluid-tightness of the fluid path is an essential requirement to ensure safety and accuracy of the delivery. Requirements are further increased by design for self-administration, which means use in a non-sterile environment, use by people without medical training, or even use by people with reduced visual or haptic capacities. The use of pre-filled syringes and cartridges, user-friendly automatic devices, modular devices with disposable modules as well as wearable devices with auto-inserters are typical solutions to improve ease of use. Reducing the number of mechanical components and design for easy and fast assembly during manufacturing are typical approaches to minimise cost.

Administering liquid medication for subcutaneous or intravenous delivery may be done using a syringe. The hollow needle of the syringe may be covered by a needle shield to prevent contamination of the medicament present in the syringe. The tightness of the connection between the needle shield and the syringe is tested using so-called container closure integrity (CCI) testing where the ingress of a contaminant into the syringe is tested. Using a syringe for the injection process may present several risks and challenges to the user or the health care professional as a manual force needs to be applied to a plunger in the syringe during an injection while holding the syringe and after the injection the needle may be exposed increasing the risk of needle sticking.

Medicament delivery devices have been developed for the delivery of a liquid medicament from a syringe or a reservoir such as a cartridge. The delivery devices may be auto injection devices such as autoinjectors or patch injectors and those have been developed to facilitate the injection process and reduce the risks for the user or health care professional using these delivery devices.

The medicament delivery devices or their subunits are adapted to accommodate a prefilled syringe including the needle shield and the medicament delivery device may require a needle shield remover that the user or health care professional removes from the device just prior to use thereby also removing the needle shield from the syringe. The needle shield remover needs to be assembled during manufacturing for obtaining the auto injection device or a subsystem e.g. a cassette or reservoir unit for a drug delivery device. The assembly of the needle shield remover with the needle shield, which is already present on the prefilled syringe, should maintain the integrity of the connection between the syringe and the needle shield ensuring CCI for the syringe in the assembled device or unit.

There is clearly a strong need for a drug delivery device which provides accurate and reliable drug delivery in a compact, easy to use, fluid-tight and robust design and which can be manufactured at low cost and which can be disassembled, separated and recycled after use. To arrive at an optimal solution, all involved components and subunits must be designed accordingly.

WO24200336 A1 describes a needle shield remover for a medicament delivery device comprising a syringe with an attached needle to the distal end covered by a needle shield, the needle shield remover comprising: a cap comprising a side wall providing a bore, the proximal section of the side wall is configured for attachment to the medicament delivery device, an elastic, tubular sleeve positioned in the bore, wherein the elastic sleeve comprises a holding section for holding the needle shield provided with an inner diameter matching the outer diameter of the needle shield, the elastic sleeve comprising a wall constructed from an elastic material, a distal end wall connecting the elastic sleeve to the cap, characterized by a passage provided in the distal end wall connecting the inner space of the elastic sleeve to the exterior, wherein, the inner dimension of the passage is greater than the outer diameter of the needle shield, wherein the elastic sleeve has a circular cross section and continuous rotational symmetry about a longitudinal axis defined by the needle. The use of a central mandrel as an expansion tool and of pressurized air flow to provide an air cushion facilitates the assembly of the needle shield in the elastic sleeve and requires a passage in the distal end wall.

WO16089864 A1 discloses a puller assembly mountable to a shield around a needle of a syringe, the shield projecting in an axial direction through an opening in a housing of a device holding the syringe, the puller assembly comprising: a grip component having a tubular body defining an interior hollow which axially receives the shield, said tubular body including opposite angular ends defining a slit that allows said tubular body to be forcibly shifted from a first arrangement to a second arrangement, said interior hollow having different sizes in said first and second arrangements, said tubular body including a radial inner face with at least one radial inward projection for directly engaging the shield, said tubular body including first and second axial ends, said first axial end adapted to abut the housing with said second axial end not in contact with the shield during manufacturing installation; and a cap grippable by a user and including a body having a surface defining a cavity, said grip component held by said cap when inserted within said cavity during manufacturing installation, wherein said at least one radial inward projection of said grip component, when said grip component is held within said cavity with the shield disposed in said interior hollow, engages the shield for removal of the shield from around the needle when the cap is moved from the housing.

WO03051423 A2 discloses a needle closure system removal device comprising a tubular member having a lower end and an upper end dimensioned to receive a shield; a base extending outwardly from said lower end of said tubular member having a generally planer lower surface; a guard extending outwardly from said tubular member spaced from said base thereby providing a gap between said base and said guard; and said base including opposing arms each having an upper surface with a generally arcuate shape providing a contoured depression surface for stabilizing said shield removal device.

WO17137366 A1 discloses a device for safe and easy removal of a rigid needle shield from a syringe, said device comprising a wall with an inner and an outer surface, the inner surface of the wall defining a tubular hollow portion along the longitudinal axis of the a rigid needle shield, said device being characterized by the fact that said inner wall comprises a recess adapted to receive a compressible gasket movable within said recess along said longitudinal axis from a portion of the recess wherein said gasket is in a relatively uncompressed state to a portion of said recess wherein said gasket is in a relatively compressed state compared to the relatively uncompressed state, wherein said recess is arranged so that the gasket grips the rigid needle shield in the compressed state.

WO15123096 A1 discloses a cap assembly mountable to a rigid shield around a needle of a syringe, said cap assembly comprising: a gripper component for directly engaging the rigid shield, said gripper component including a support frame and a liner, said support frame defining an interior hollow, said liner secured to said support frame within said interior hollow to provide a gripping section that defines a bore sized to axially receive the rigid shield, said liner formed of a material that is resilient relative to a material forming said support frame and is deformable when sandwiched between said support frame and the rigid shield; and a base cap grippable by a user and comprising a body, said body including a surface defining a cavity for accommodating said gripper component, said body surface configured to compress said gripper component from a first arrangement to a second arrangement when said gripper component inserts within said cavity during manufacture, wherein said liner, when said gripper component is disposed in said first arrangement, does not grip for shield removal purposes the rigid shield when disposed in said bore, and wherein said liner, when said gripper component is disposed in said second arrangement with the rigid shield disposed in said bore in any rotational orientation relative thereto, is held by the support frame so as to be deformed against and grip the rigid shield for shield removal purposes so as to allow for removal of the rigid shield from around the needle.

WO 06061354 A1 discloses a needle cap remover for a needle cap of a drug delivery device, comprising: - a body having a material which differs from the material of the needle cap or a rigid needle shield encasing the needle cap in at least one parameter, - wherein a body portion, which covers the needle cap or the rigid needle shiel encasing the needle cap, is adapted to capture the needle cap or the rigid needle shield encasing the needle cap by a positive and/or non-positive connections before or during removing of the needle cap.

WO20245206 A1 discloses an apparatus for removing a needle shield, comprising a needle unit comprising a needle arranged along a first axis, a needle shield which covers the needle , a main part, a connector for connecting the needle shield to the main part by a positive connection or an adhesive bond, such that the needle shield is removed when the main part is moved along the first axis away from the needle.

WO19074788 A1 discloses a needle shield puller device for a drug delivery system for removing a protective shield, the device comprising a cup shaped body defined about a longitudinal axis, said body having a sidewall circumscribing an interior space and a distal bottom wall, and a proximal opening in communication with the interior space, wherein the proximal opening is configured to receive the protective shield to be disposed within the interior space, wherein the sidewall includes a plurality of sidewall openings formed therein, a plurality of fingers each having a proximal end flexibly coupled with the sidewall and extending distally within the corresponding sidewall openings, each of the fingers further including at least one projection extending in a circumferential direction and projecting toward the interior space, wherein the at least one projection on each finger is engageable with the protective shield when the protective shield is disposed within the interior space.

WO18202459 A1 discloses a needle shield remover comprising a generally tubular body having a longitudinal extension, - a grip element comprising a braided sleeve arranged to said body, said braided sleeve having an opening with such a diameter that a needle shield may be introduced, and wherein said braided sleeve has such a diameter that a friction fit is created between said braided sleeve and said needle sleeve when said needle cover is pulled for removing said needle cover..

EP3257540 A1 discloses a cap assembly for a medicament delivery device, the cap assembly comprising: a cap body arranged to be connected to the medicament delivery device for protecting and for removing a medicament delivery member shield, which cap body has an inner cap structure defining a channel extending along the central axis of the cap body, and a gripping member configured to be received in the channel with a friction fit and to receive a medicament delivery member shield, wherein the gripping member has a first leg, a second leg, and an transverse portion extending between the first leg and the second leg, which transverse portion defines the leading edge of the gripping member when received by the channel, each of the first leg and the second leg having a respective proximal portion extending distally from the transverse portion and extending parallel to each other, the first leg and the second leg also having a respective distal portion, the distal portions being radially flexible and extending away from each other to allow the gripping member to receive a medicament delivery member shield between the distal portions, each distal portion being provided with a medicament delivery member shield gripper configured to engage with a medicament delivery member shield when the gripping member is pushed into the channel and the distal portions flex radially inwards.

WO23224597 A1 discloses a syringe capping and uncapping device comprising: a body portion extending from a bottom end to a top end along a longitudinal axis, the body portion comprising a cavity configured to receive and hold a cap of a syringe; an annular flange extending radially from the top end of the body portion, the annular flange surrounding an opening that provides a passageway into the cavity of the body portion; wherein the body portion comprises a first portion formed from a resilient material and a second portion formed from a rigid material, the second portion circumferentially surrounding the first portion; and wherein at least a portion of the body portion is configured to be compressed inwardly towards the longitudinal axis to apply pressure onto the cap of the syringe that is positioned in the cavity while the syringe is pulled axially to remove the cap from the syringe.

WO23198567 A1 discloses a cap for removing a needle shield attached to a medical container provided with an injection needle, the cap including: a tubular body extending along a longitudinal axis A, the tubular body having a distal end and an opposite proximal end for attachment to a bottom housing of an autoinjector, an inner sleeve proximally extending inside the tubular body from said distal end, the inner sleeve defining an inner cavity for receiving the needle shield, the inner sleeve having clamping means for clamping the needle shield when the needle shield is inserted inside the inner cavity, the clamping means including an axial abutment surface for abutting against a proximal end of the needle shield, a plug at least partially extending inside the inner cavity, the plug having a distal end portion, a proximal end portion and an intermediate portion extending between the distal end portion and the proximal end portion, wherein at least the proximal end portion of the plug is movable between a first position, in which the proximal end portion allows the proximal end of the needle shield to distally pass the axial abutment surface of the clamping means, and a second position, proximal to the first position, in which the proximal end portion of the plug moves in the proximal direction to push the needle shield back towards the axial abutment surface.

WO22192310 A1 discloses a drug delivery device comprising: a housing having an opening and a longitudinal axis; a syringe including a needle having an insertion end configured to extend at least partially through the opening during a delivery state; and a syringe holder comprising: a proximal end and a distal end, a hollow interior configured to receive at least a portion of the syringe, a side opening distal to the proximal end, and an outwardly extending protrusion proximal to the side opening, wherein during assembly of the syringe holder into the housing the outwardly extending protrusion is configured to support the syringe holder at a first axial position with respect to the longitudinal axis of the housing.

WO20070260 A1 discloses a cap remover, comprising: an outer member; an activation member at least partially received within the outer member and configured to move relative to the outer member; and an inner member positioned within the outer member and configured to radially compress under an axial pressure force asserted by the activation member that moves the activation member from a first axial position to a second axial position, such that the inner member compresses from a first position to a second position.

EP3946521 A1 discloses an injection device with a longitudinal axis comprising: -a housing for holding a product container, wherein the product container has a firmly connected injection needle, wherein a needle protection cap is arranged on the product container in a detachable manner, which encloses the injection needle and seals it sterilely from the environment, -a cap, which is separable at a distal end of the housing, wherein the cap comprises an engagement element in order to cause the removal of the needle protection cap from the product container when removing the cap from the injection device, wherein the engagement element is deformable in such a way that the engagement element is radially spaced from a spaced position in which the engagement element is radially spaced from the needle protection cap to an engagement position, in which the engagement element is engaged with the needle cap, wherein the engagement element is deformed when the cap is removed, characterized in that the cap comprises a sleeve element with a recess with a distal and a proximal edge, wherein the engagement element in the spaced position is at least partially undeformed by the distal edge of the recess, is deformed or radially deformed outwards and wherein the engagement element is at least partially undeformed, deformed or radially deformed inwards in the engagement position by the proximal edge of the recess.

WO17139377 A1 discloses a needle shield puller for removing a needle shield having a needle shield diameter from a syringe having a needle and a longitudinal axis, the needle shield puller comprising: a deformable, generally tubular body having a proximal end with a proximal opening, a distal end, and an opening extending from the proximal opening toward the distal end, the opening having an opening diameter and being shaped to accommodate at least a portion of the needle shield, the body having an initial state and a tension state, the body being configured such that the opening diameter is greater in the initial state than in the tension state; a cinch secured to the body at the proximal opening to secure the needle shield puller to the needle shield in a mounted configuration, the cinch having a cinch diameter, the cinch biasing the cinch diameter to a relaxed cinch diameter, the relaxed cinch diameter being less than the needle shield diameter; a handle at a distal end of the body, the handle being graspable to allow a user to exert a tensile force on the body and place the body in the tension state; and an installation aid comprising an arm releasably engageable with the cinch and the proximal opening, the installation aid graspable by the user for pulling in a direction radially away from the longitudinal axis in a mounted configuration to expand the cinch diameter so that the cinch diameter is greater than the needle shield diameter.

WO17098437 A1 This disclosure relates to a medical injector comprising: an injector housing defining a reservoir; a plunger rod; a stopper engaged with a portion of the plunger rod and slidably disposed within the reservoir, the stopper sized relative to an interior of the injector housing to provide sealing engagement with a sidewall of the injector housing; a needle having a sharpened first end and a second end in communication with the reservoir; a first cap covering the sharpened first end of the needle; and a second cap covering at least a part of the first cap, the second cap comprising a body having a proximal end, a distal end, and a hemispherical member including a gripping component, wherein the second cap is engaged with the first cap such that removal of the second cap simultaneously removes the first cap from the first end of the needle.

EP3047868 A1 discloses a recoil reducing needle shield for covering a drug container with a needle, comprising: an outer shell, including an upper portion, a lower portion, and at least one flexible interface positioned therebetween and defining a fulcrum therebetween, such that radially inward flexing of the upper portion causes radially outward flexing of the lower portion about the at least one flexible interface; and an inner cover including an open proximal end, a closed distal end, and a needle cavity defined therein for receiving a portion of the drug container including the needle, at least a portion of the inner cover being disposed within the outer shell; wherein the open proximal end of the inner cover is coupled to at least a portion of the lower portion of the outer shell, such that radially outward flexing of the lower portion stretches the open proximal end of the inner cover, thereby reducing an initial pull-off force required to remove the needle shield from the drug container.

US4430082 A discloses an improved hypodermic syringe assembly of the type comprising: (a) a medication chamber having an interior for containing liquid and first and second ends; (b) a needle, attached to the first end of said medication chamber, through which liquid is expelled from said chamber; (c) an elastomeric stopper, movably disposed within said medication chamber, movement of said stopper from said second end of said chamber toward said first end serving to expel liquid from said chamber through said needle; (d) a first protective sleeve into which said needle is removably embedded; and (e) a second protective sleeve which removably covers said first protective sleeve and, in turn, said needle, said second protective sleeve having means at one end thereof for attachment to said stopper, whereby said second protective sleeve and said stopper when so attached serve as a plunger; the improvement in said syringe assembly comprising the provision in said second protective sleeve of flexible means for gripping said first protective sleeve, whereby simultaneous removal of both said first and second protective sleeves from said needle are effected by removing said second protective sleeve from over said needle while inwardly deflecting said flexible gripping means in order to grip said first protective sleeve.

US4742910 A discloses a needle sheath holder for use with a medical needle assembly which includes a base and a needle mounted thereon, wherein a hollow needle sheath member is telescoped over said needle and frictionally engaged with said base when said needle is not being used as a conduit for fluid, a needle shaft holder comprising a hollow barrel member having an open upper end, a shield member on said barrel member spaced from said open end, and gripper members in said open end shaped to frictionally engage a needle sheath member inserted into said barrel member through said open end so as to grip and retain the needle sheath member in said barrel member.

It is an objective of the invention to provide an improved drug delivery device which is accurate, reliable, easy to use and cost effective, overcoming the drawbacks of or introducing alternatives to the prior art. Several aspects or variants of the present invention contribute to an overall optimum including an improved ease of separation of the used materials and components after use. The aspects or variants are applicable to all sorts of reusable, semi-reusable or disposable drug delivery devices such as auto injectors, pen injectors, auto pen injectors, safety injectors or safety syringes.

It is an additional objective to provide improved apparatuses and assembly methods for the drug delivery device as disclosed in the corresponding claims and the description.

### DEFINITIONS

The term "substance", "drug", "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The distal end or distal direction is defined by the direction of the needle configured to penetrate the skin of the patient. For an injection pen this may be the injection needle and the end of the pen holding the needle or being configured to hold the needle is the distal end. For an infusion device the distal end and the distal direction is towards the needle configured to penetrate the skin of the patient, which may be along the axis of the device or tilted or perpendicular to the axis of the device. The distal direction in an infusion device represents the direction in which the medicament flows towards the insertion needle. The proximal direction or end is opposite to the distal direction or end.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours. If not explicitly mentioned otherwise, the term "pump" is referring to an infusion system.

In the claims, the words "comprising" or "including" do not exclude other elements or steps, and the indefinite article "a" or "an" does not exclude a plurality. For example, "sealing" or "protrusion" does not exclude the fact that there may be two "sealings" or "protrusions" that functionally or structurally fulfil the purpose of "a sealing" or "a protrusion".

### DESCRIPTION OF THE INVENTION

In a first aspect of the present invention, a cassette assembly or cassette unit for an improved operation stability of a drug delivery device is described. The cassette unit facilitating ease of use and reliability of a modular or integral injection system and ease of assembly of a syringe and a cap.

The objectives are solved according to the invention by a cassette unit for an injection device, the cassette unit comprising: - a sleeve defining a longitudinal axis with a proximal opening, - a holder integrated coaxially in the sleeve and prepared to accommodate a syringe comprising a barrel, a flange, a stopper, a needle and a shield, - a latch situated near the proximal opening on the inner wall of the sleeve or on the inner wall of the holder, - a cap removably attached to the distal end of the sleeve e.g. by a snap fit , - a proximally open tube coaxially with the longitudinal axis, the tube having a wall or shroud defining a hole and having a distal cover, in other words the tube defines a bore having a distal lid or the tube defines a cavity or an interior space, where the tube is constructed from an elastic material and the wall forms at least three bores distributed around the circumference of the wall or shroud, each bore running parallel to the longitudinal axis and piercing the distal cover. In other words an elastic shroud with a cavity adapted to grip a shield e.g. a RNS and with at least three orifices or at least two slits circumferentially distributed outside the shroud and adapted to be engaged by a shroud enlarging tool.

This arrangement of the bores and of the wall allows for frictionless and fast placement of the cassette into a manufacturing apparatus providing capabilities to prepare the tube for the introduction and fastening of a syringe in the cassette and afterwards easy removal of the cassette from the apparatus.

A finished cassette unit according to the first aspect of the invention may for example be inserted into a drive unit of a reusable or disposable auto injector.

Further the cassette unit for an injection device according to the invention may include the tube integrated in or forming the distal end of the cap. This eases the manufacturing process of the cassette assembly and improves the kinematic arrangement of the cap for easy shield removal. Further the cap and the tube may be molded or formed integrally from one single elastic material. This eases the separation of the materials after use of the device or the unit for sustainable recycling.

The wall of the tube may be reinforced around and/or along the bore. The wall or shroud may include a rotationally closed surface, or other enveloping structure with a continuous rotational symmetry and circular cross-section. The wall or shroud may be reversibly elastically deformable. The wall may be constructed from a woven structure including openings providing a braided structure. The wall may be provided with a closed surface as a monolithic component constructed from an elastic material. This may reduce material consumption and adapts for tolerances of the prefilled syringe configuration.

The distal end of the elastic sleeve may be connected or connectable to an inner surface of the end wall. The inner surface of the end wall faces in the proximal direction whereas the outer surface of the end wall faces the distal direction. The cap with the tube may be produced by multi-component injection molding, for example two-component injection molding of two different thermoplastic polymers for the cap and for the elastic sleeve whereby an elastomer is selected for the elastic sleeve. The cap may be injection molded first followed by molding the elastic sleeve or, vice versa, the tube is injection molded first followed by injection molding of the cap. Multi-component injection molding may provide for easy manufacturing using less separate parts such as the metal parts of the prior art.

The wall or shroud of the tube may be constructed from a highly stretchable material, such as a silicone rubber (LSR), a thermoplastic elastomer (TPE) or a thermoplastic polyurethane (TPU). The cap with the tube may be produced using injection molding of three or four different polymers. The cap or the tube may be constructed from a thermoplastic biopolymer, such as, for example bio-polyethylene or bio-polypropylene. As an alternative, the cap or the tube may be made from a recycled polymer such as recycled polyethylene or recycled polypropylene. Using organic or biopolymers or recycled polymers may reduce the environmental impact or carbon footprint of the device. As an alternative, the cap may be made from a biodegradable polymer. As another alternative, the cap may be made from a fossil-based or plant-based polymer.

The wall or shroud may form two, three, four or five bore with a circular, C or I shaped cross section, the bore distributed around the circumference of the wall or shroud. Such the wall or hole may expand from a circular into a rectangular or polygonal cross section to receive the shield of the syringe.

The distal cover of the tube may form a flange. The tube may be provided as a circular cylindrical sleeve with the distal cover and the flange that depends radially or extends radially outward from the distal end or the distal cover of the sleeve. As an alternative, the cylindrical sleeve and /or the flange may have a rectangular or triangular or polygonal shaped cross section. This allows for secure connection with the cap and the shield.

The hole and the wall of the tube may be adapted to firmly receive at least a part of the shield of the syringe in a holding section due to the elastic properties of the wall. The wall may include rippled or corrugated or zig-zag structures. In an embodiment, the wall, preferably the inner surface of the holding section configured for engaging the shield, is roughened or textured. This may improve the friction-fit between the inner surface of the holding section and the outer surface of the needle shield and provides improved rigidity and good functional stability when temperature changes and within a prolonged shelf time.

Further the cassette unit for an injection device according to the invention may include a central opening in the distal cover of the tube, the central opening formed as an orifice or as an aperture or as a slit. This allows for axial introduction of a tool e,g. of a pin designed to support a distal end of a shield of a syringe and/or for pressure compensation or deairing.

Within the scope of the invention as a second aspect an apparatus or in other words a shroud enlarging tool is presented to assemble a syringe in a cassette unit for an injection device, the apparatus including: - a base having a proximal surface and forming a bushing, the bushing defining a or arranged along the longitudinal axis, - a support guided coaxially in a trench formed in the proximal surface of the base, the support biased in proximal direction by a spring, - a ram axially guided and moveable in the bushing, the ram forming a circumferential fulcrum on its proximal end section, - a cylindrical shaft mounted fixedly to the base and proximally spaced above the support, the shaft having a proximal opening to accommodate the distal end of the cassette unit and having a distal surface, - at least three spreaders distributed around the longitudinal axis between the support and the shaft, each spreader having a beam radially running to the longitudinal axis with its inner end bearable on the fulcrum, the beam urged by the support towards the distal surface of the shaft and the outer end of the beam projecting radially beyond the shaft, each spreader having further a mandrel perpendicularly attached to the inner end of the beam, the mandrel in a first position of the spreader is running in proximal direction parallel to the longitudinal axis, - a pusher coaxially guided on the shaft, the pusher able to move the outer end of the beam in distal direction, where due to the distal movement of the pusher, the beam pivots in the fulcrum and the support is urged distally into the trench against the force of spring and the mandrel is inclined in radial direction with respect to the longitudinal axis in a second position of the spreader. More generally the beam is pivotable in the fulcrum and the support is urgeable distally into the trench against the force of the spring and the mandrel is inclinable or moveable in a radial outward direction with respect to the longitudinal axis. In variant embodiments one or more of the apparatuses according to the invention are driven, manipulated and operated on a separate hub or integrated in a drug device product final assembly line or in a fill-finish robotic cell. For instance, the base of the apparatus is fixed to the machine bed of an assembly line and the pusher, the ram and the pin may be manipulated by servo drives or gearings of the assembly line and its control computer to e.g. perform the steps of a method according to the invention, where on the assembly line a syringe is assembled in a cassette unit using the apparatus.

The apparatus according to the invention, where a movement of the ram in distal direction loosens the inner end of the beam from the fulcrum and enables the spreader to move radially inwards. Due to the freely maneuverable spreader removing a cassette from the apparatus is eased.

The apparatus according to the invention, where a movement of the ram in proximal direction catches the inner end of the beam into the fulcrum, where due to the force of spring biasing the support in proximal direction each spreader pivots in the fulcrum of the ram towards or to its first position. Such in an apparatus with no cassette inserted each mandrel runs parallel to and all mandrels are arranged circumferentially with the longitudinal axis. Such each mandrel is aligned to a corresponding bore in the wall of a tube in a cassette and is prepared to receive a cassette.

The apparatus according to the invention, where the surface of the mandrel is constructed from or coated with a low-friction compound. This allows for easy insertion and removal of a cassette to and from the apparatus and promotes the maneuverability of the spreader with the mandrel while removing a cassette from the apparatus. Further the cross section of the mandrel may be circular, C or I shaped.

Further the apparatus to assemble a syringe in a cassette unit for an injection device according to the invention may include a pin running along the longitudinal axis and guided axially in the ram, the proximal end of the pin designed to support a distal end of a shield of a syringe.

Within the scope of the present invention as a third aspect, a method is presented to assemble a syringe in a cassette unit for an injection device according to the invention and carried out on, in or by the apparatus according to the invention, the method including the steps of:
- initializing the apparatus with no cassette inserted by moving the pusher and the ram each in a proximal position, whereby due to the force of spring biasing the support in proximal direction each spreader pivots in the fulcrum of the ram to its first position, wherein each mandrel runs parallel to and circumferential with the longitudinal axis. Such each mandrel is alignable to a corresponding bore in the wall of a tube in a cassette.
- aligning mandrel and bore coaxially by rotating a cassette unit about the longitudinal axis and/or by means of axial guiding structures formed complementary on both the cassette unit and the apparatus.
- inserting a cassette unit coaxially by the opening of the apparatus whereby the mandrel of each spreader slides into the corresponding bore in the wall. Due to the alignment of mandrel and bore the friction in between is minimized and the placement of the cassette unit is rotationally and coaxially defined with respect to the apparatus. The alignment may be improved by using axial guiding structures complementary formed on both the cassette unit and the apparatus. The cassette unit may be inserted in distal direction into the opening until it finds an axial limit stop formed on or by the shaft or the base or the spreader.
- stretching the wall by moving the pusher in a distal position to urge each spreader in its second position whereby the wall with the bore is circumferentially stretched due to the inclined mandrel. This radially widens the hole defined by the wall to a funnel-like structure.
- inserting a syringe coaxially by the proximal opening of the cassette unit into the holder until the flange of the syringe has passed the latch on the inner wall of the sleeve or on the inner wall of the holder whereby the shield is placed within the radially expanded hole defined by the stretched wall. The placement of the shield performs without any axial or radial forces urging the shield relative to the syringe due to the play between the widened hole and the shield. Such CCI is not compromised.
- shrinking the wall by removing or releasing the ram from its distal position and/or the pusher and from its proximal position to free the outer end and/or the inner end of the beam of each spreader whereby the elastic relaxation of the stretched wall declines the mandrel of each spreader back towards the longitudinal axis and firmly shrinks at least parts of the wall circumferentially onto at least parts of the outer surface of the shield of the syringe. Additionally, the proximally directed bias force of the spring to the support helps to decline the mandrel of each spreader back towards the longitudinal axis.
- removing the cassette unit in proximal direction from the apparatus whereby the mandrel of each spreader is retracted from its bore and each spreader is free to move radially. Due to the freely maneuverable spreader pulling the mandrel from the bore and removing the cassette from the apparatus is eased.

The method according to the invention may further include the steps of:
- initializing the apparatus by moving the proximal end of pin into a distal position, wherein the proximal end of the pin forms an axial limit stop for the distal end of the shield of the syringe.
- inserting a syringe until the distal end of the shield abuts the proximal end of pin i.e. the limit stop within the hole whereby a proximal section of the pin passes the opening in the cover of the tube or the cap. This allows the syringe with the shield to be installed in the cassette unit at a defined axial position.
- moving the pin in proximal direction synchronously with the distal end of the shield of the syringe while removing the cassette unit until the mandrel of each spreader is retracted from its bore completely. Such any distally directed force acting on the shield while removing the cassette unit does not induce a relative movement of the shield and the rest of the syringe.

The steps of the above methods according to the invention may be carried out by one or more apparatuses driven, manipulated and operated on a separate hub or integrated in a drug device product final assembly line or in a fill-finish robotic cell. For instance, the base of the apparatus is fixed to the machine bed of an assembly line and the pusher, the ram and the pin may be manipulated by servo drives or gearings of the assembly line and its control computer to e.g. perform the steps of a method according to the invention, where on the assembly line a syringe is assembled in a cassette unit using the apparatus carrying out computer controlled steps of the method.

### BRIEF DESCRIPTION OF THE DRAWINGS

The several aspects of subject matter of the invention will be explained in more detail in the following text with reference to preferred exemplary embodiments which are illustrated in the attached drawings, in which:
- Figs. 1a to 4b: refer to a cassette unit embodiment according to the invention:
- Fig. 1a: depicts a lateral view of a cassette unit;
- Fig. 1b: depicts a longitudinal cut of a cassette unit;
- Fig. 1c: depicts a perspective view of a cassette unit;
- Fig. 1d: depicts a partial longitudinal cut in perspective view of a cassette unit;
- Fig. 2a: depicts a proximal view of a cap with a tube;
- Fig. 2b: depicts a lateral view of a cap with a tube;
- Fig. 2c: depicts a distal view of a cap with a tube;
- Fig. 2d: depicts a perspective view of a cap with a tube;
- Fig. 3a: depicts a proximal view of a tube;
- Fig. 3b: depicts a lateral view of a tube;
- Fig. 3c: depicts a distal view of a tube;
- Fig. 3d: depicts a perspective view of a tube;
- Fig. 4a: depicts a lateral view of a cassette unit with an assembled syringe;
- Fig. 4b: depicts a longitudinal sectional view of a cassette unit with an assembled syringe;
- Figs. 5a to 5d: refer to an apparatus embodiment according to the invention:

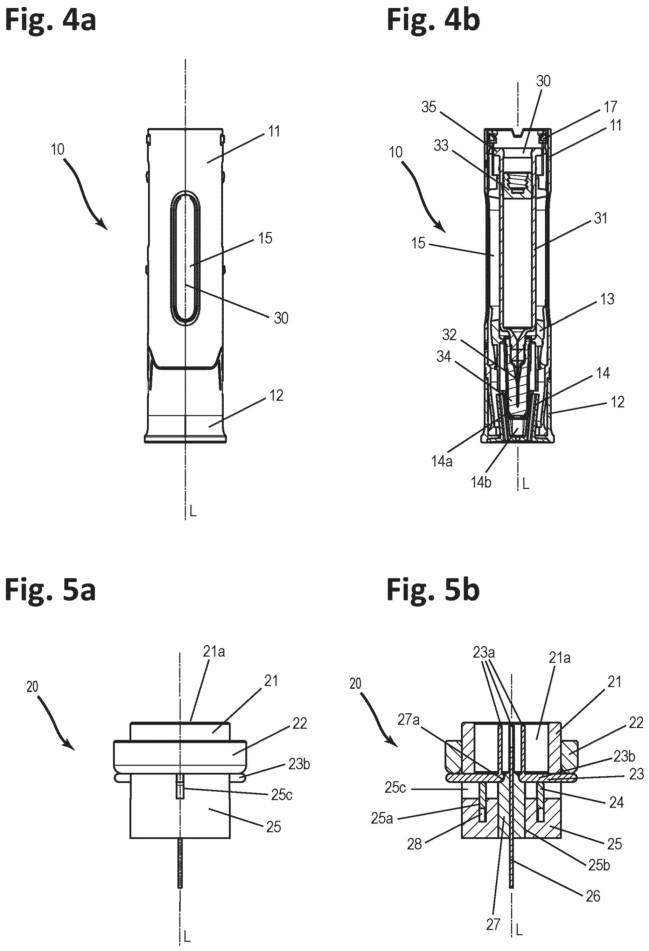

- Fig. 5a: depicts a lateral view of an apparatus;
- Fig. 5b: depicts a longitudinal sectional view of an apparatus;

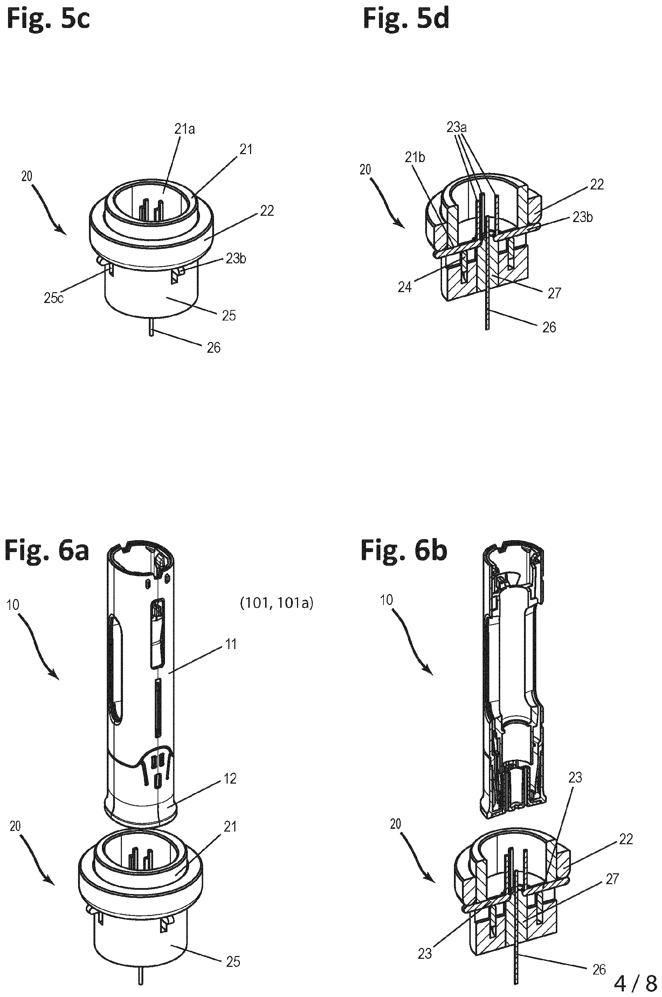

- Fig. 5c: depicts a perspective view of an apparatus;
- Fig. 5d: depicts a longitudinal cut in a perspective view of an apparatus;
- Figs. 6a to 6p: refer to an exemplary method according to the invention:
- Fig. 6a: depicts a perspective view of a cassette unit and an apparatus in a step of initializing the apparatus;
- Fig. 6b: depicts longitudinal cuts in a perspective view of a cassette unit and an apparatus in a step of initializing the apparatus;

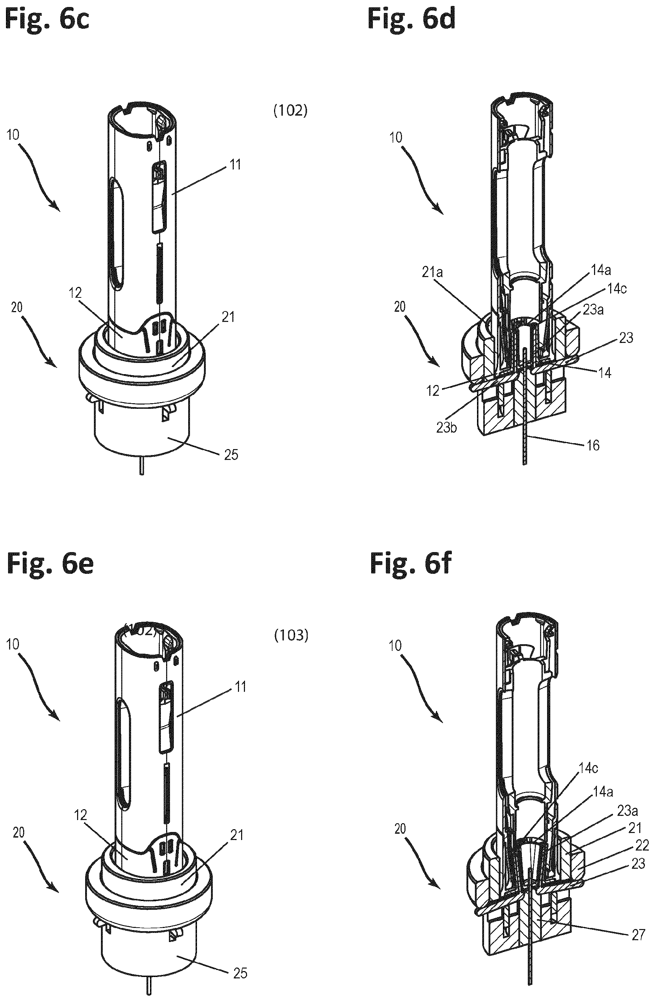

- Fig. 6c: depicts a perspective view of a cassette unit and an apparatus in a step of inserting the cassette unit in the apparatus;
- Fig. 6d: depicts longitudinal cuts in a perspective view of a cassette unit and an apparatus in a step of inserting the cassette unit in the apparatus;
- Fig. 6e: depicts a perspective view of a cassette unit and an apparatus in a step of stretching the wall of the tube;
- Fig. 6f: depicts longitudinal cuts in a perspective view of a cassette unit and an apparatus in a step of initializing the apparatus;

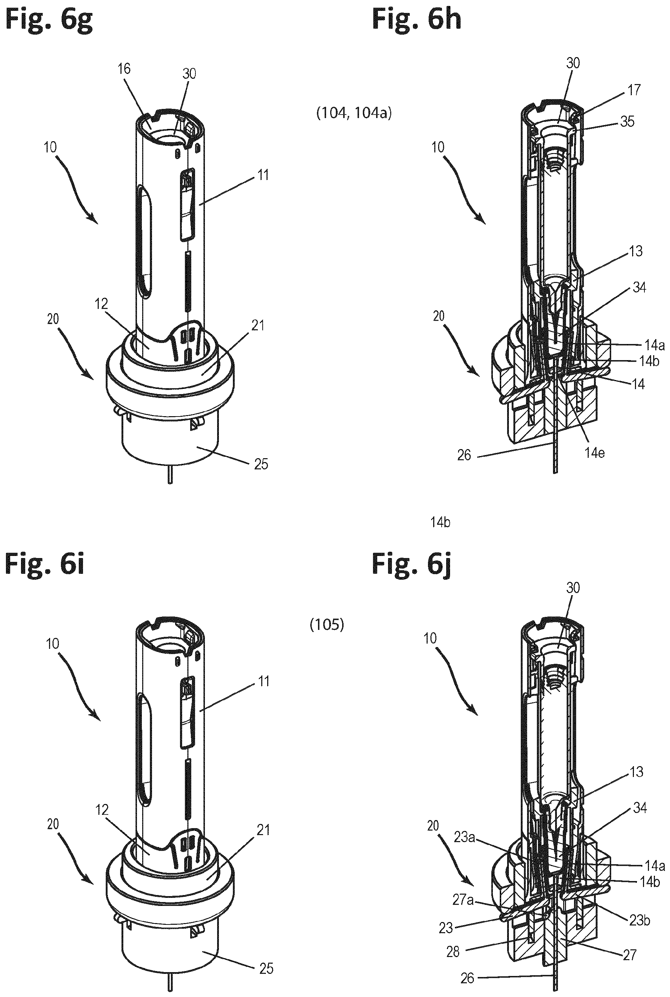

- Fig. 6g: depicts a perspective view of a cassette unit and an apparatus in a step of inserting the syringe into the cassette unit;
- Fig. 6h: depicts longitudinal cuts in a perspective view of a cassette unit and an apparatus in a step of inserting the syringe into the cassette unit;
- Fig. 6i: depicts a perspective view of a cassette unit and an apparatus in a step of shrinking the wall of the tube;
- Fig. 6j: depicts longitudinal cuts in a perspective view of a cassette unit and an apparatus in a step of shrinking the wall of the tube;

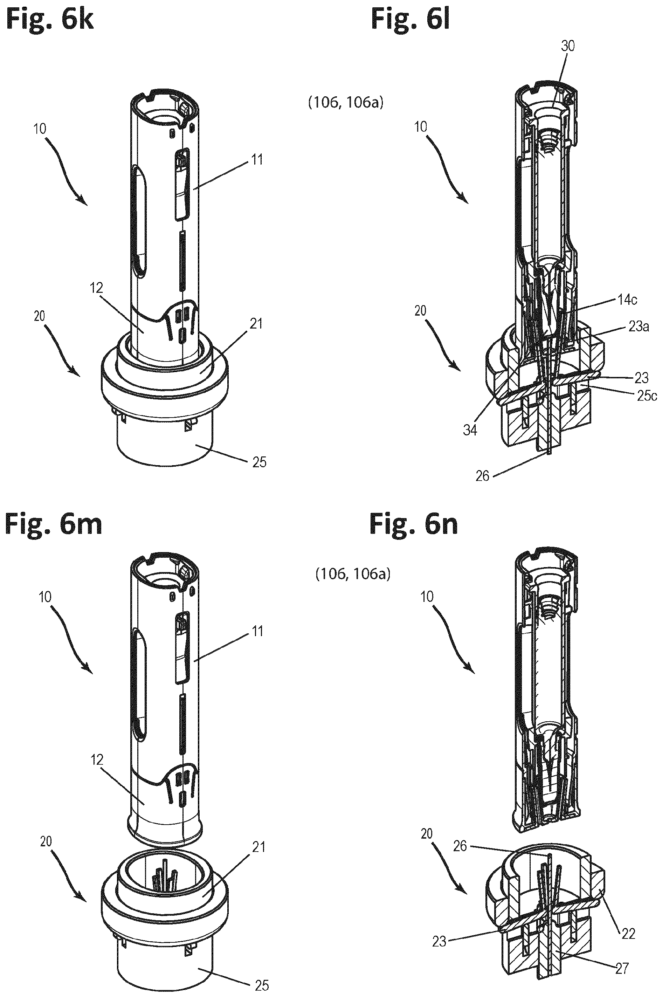

- Fig. 6k: depicts a perspective view of a cassette unit and an apparatus with the cassette unit removed from the apparatus;
- Fig. 6l: depicts longitudinal cuts in a perspective view of a cassette unit and an apparatus in a step of removing the cassette unit from the apparatus;
- Fig. 6m: depicts a perspective view of a cassette unit and an apparatus with the cassette unit removed from the apparatus;
- Fig. 6n: depicts longitudinal cuts in a perspective view of a cassette unit and an apparatus with the cassette unit removed from the apparatus;

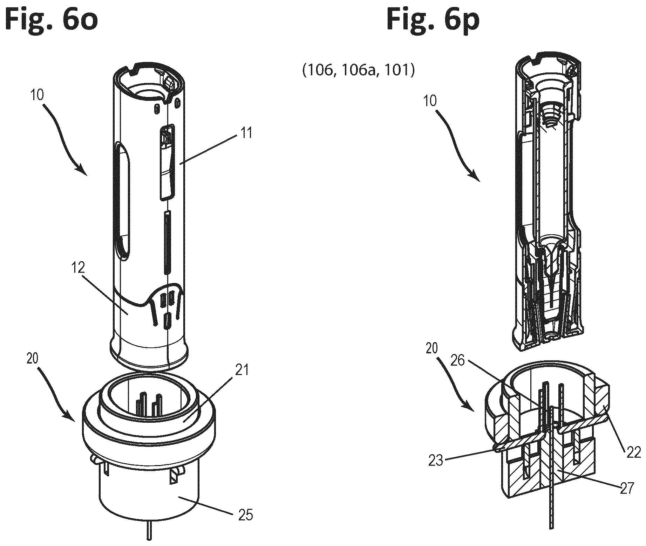

- Fig. 6o: depicts a perspective view of a cassette unit and an apparatus with syringe assembled in the removed cassette unit and the apparatus initialized again;
- Fig. 6p: depicts longitudinal cuts in a perspective view of a cassette unit and an apparatus with the syringe assembled in the removed cassette unit and the apparatus initialized again;

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

As outlined in the introductory paragraphs the present invention has several aspects and variants, all contributing to an overall optimum of an improved drug delivery device which is accurate, reliable and easy to use while still suitable for conducting complex therapies at as low cost as possible. While most preferred embodiments are explained using the example of a cassette unit and an apparatus as shown in figures 6a for example it is evident, that the same aspects and variants of the current invention may also be used to improve any other kind of drug delivery devices or assembly and/or fill-finish processes thereof, wherever a comparable requirement or improvable feature is present.

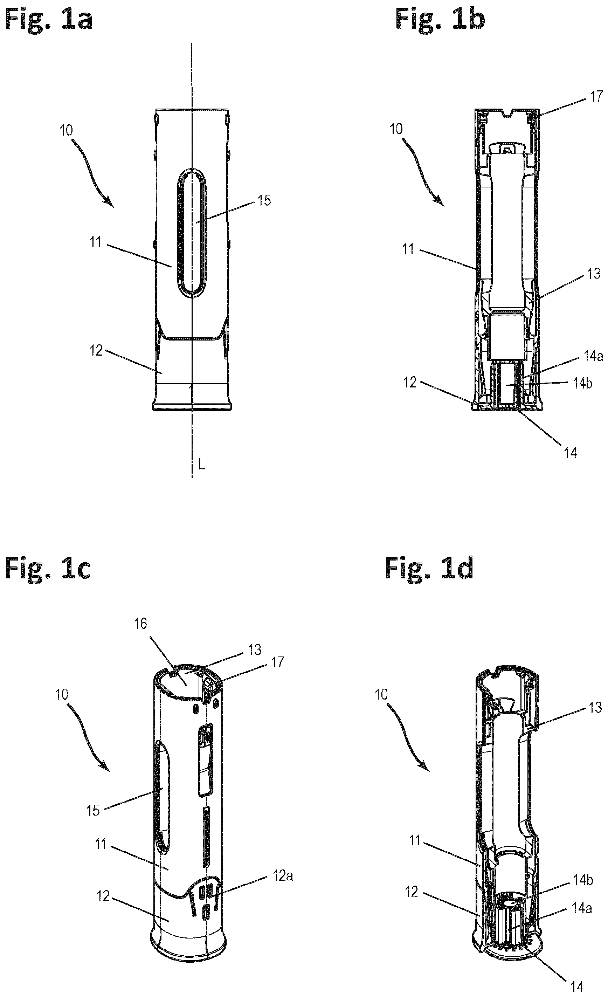

Figures 1a to 1d refer to a cassette unit embodiment according to the invention and depict a lateral view, a longitudinal cut view, a perspective view and a partial longitudinal cut in perspective view of a preferred embodiment of a cassette unit 10 according to the present invention. The cassette unit 10 includes a sleeve 11, a holder 13, a cap 12 and a tube 14. The holder 13 is coaxially inserted in the sleeve 11 and is prepared to accommodate a syringe coaxially along a longitudinal axis L. The sleeve 11 and the holder 13 open a window 15 to facilitate sight to the inside. An opening 16 is formed coaxially with the longitudinal axis L on the proximal side of the sleeve 11. A latch 17 is provided to secure a once inserted syringe in position. The cap 12 is attached removeable to the distal side of the sleeve 11 and secured to the sleeve 11 by a snap fit 12a. The tube 14 is fitted in the cap 12 and opens a hole 14b coaxially with the longitudinal axis L on the proximal side of the tube 14. The hole 14b is defined by an inside of a wall 14a of the tube 14.

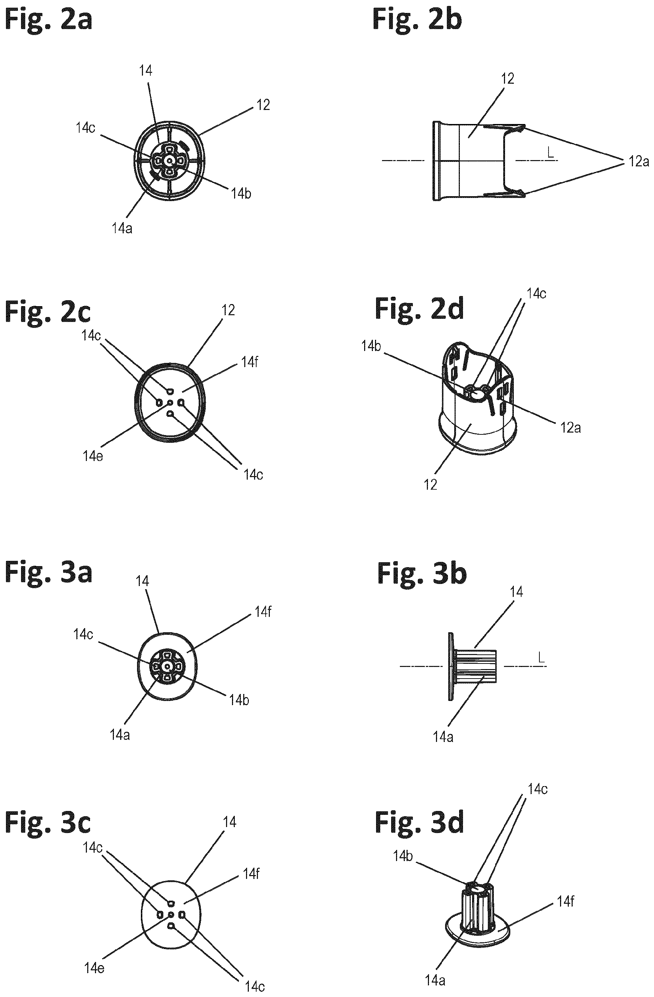

Figures 2a to 2d refer to a cap embodiment according to the invention and depict a proximal view, a lateral view, a distal view and a perspective view of a preferred embodiment of a cap 12 according to the present invention. The cap 12 is adapted to be removable attachable to the distal side of a cassette unit 10 as shown in figures 1a to 1d. The cap 12 contains the tube 14 with the wall 14a, the wall 14a defining a hole 14b. Four bores 14c are formed in the circumference of the wall 14a. The bores 14c running parallel equidistant to the longitudinal axis L. A flange 14f is formed as a distal end of the tube 14. The flange is affixed or builds the distal end of the cap 12. The four bores 14c pierce the flange 14f from the distal side and lead into the wall 14a. A central opening 14e is formed in the flange 14f to allow a tool insertion into the hole 14b and/or deairing of the hole 14b. Two tongues are formed on the side wall of the cap 12 showing the snap fit 12a.

Figures 3a to 3d refer to a tube embodiment according to the invention and depict a proximal view, a lateral view, a distal view and a perspective view of a preferred embodiment of a tube 14 according to the present invention. The tube 14 with the wall 14a defines a hole 14b and constructed from an elastic material. Four bores 14c are formed in the circumference of the wall 14a. The bores 14c running parallel equidistant to the longitudinal axis L. A flange 14f is formed as a distal end of the tube 14. The four bores 14c pierce the flange 14f from the distal side and lead into the wall 14a. A central opening 14e is formed in the flange 14f to allow a tool insertion into the hole 14b and/or deairing of the hole 14b.

Figures 4a and 4b refer to a cassette unit embodiment according to the invention and depict a lateral view and a longitudinal sectional view of a preferred embodiment of a cassette unit with an assembled syringe according to the present invention. The cassette unit 10 includes a sleeve 11, a holder 13, a cap 12 and a tube 14 and an inserted syringe 30. The syringe 30 includes a barrel 31, a staked needle 32, a stopper 33, a shield 34 and a flange 35 formed as or on a proximal end of the barrel 31. The holder 13 is coaxially inserted in the sleeve 11 and accommodates the syringe 30 coaxially along a longitudinal axis L. The sleeve 11 and the holder 13 open a window 15 to facilitate sight to a barrel 31 of the syringe 30. A latch 17 is provided and secures the inserted syringe 30 in position. The cap 12 is attached removeable to the distal side of the sleeve 11 and secured to the sleeve 11 by a snap fit 12a. The tube 14 is fitted in the cap 12 and opens a hole 14b coaxially with the longitudinal axis L on the proximal side of the tube 14. The hole 14b is defined by an inside of a wall 14a of the tube 14. The wall 14a is tightly shrunk to a distal section of the outer surface of the shield 34.

Figures 5a to 5d refer to an apparatus embodiment according to the invention and depict a lateral view, a longitudinal sectional view, a perspective view and a longitudinal cut in perspective view of a preferred embodiment of an apparatus 20 according to the present invention. The apparatus 20 includes a shaft 21, a pusher 22, a spreader 23, a support 24, a base 25, a pin 26, a ram 27 and a spring 28. A common longitudinal axis L is defined by the shaft 21 and its proximal opening 21a and the base 25 and its bushing 25b. The shaft 21 is formed as a sleeve and guides the pusher 22 coaxially on its outer surface. The pusher 22 has the form of a ring or a sleeve and is axially moveable. The base 25 forms four slits 25c running evenly distributed radially from the central bushing 25b to the outside. In the proximal surface of the base 25 a circular coaxial trench 25a is formed. The trench accommodates the spring 28 and the support 24. The support 24 is guided and axially moveable in the trench and biased in proximal direction by the force of the spring 28. The ram 27 is guided and axially movable in the bushing 25b. The ram 27 is formed as round cylinder forming a fulcrum 27a on its proximal end section. The fulcrum 27a is prepared to bear the four spreader 23 by the inner end of their beam 23b for a pivotal movement. The four spreaders 23 sit on the proximal rounded top of the support 24 and are axially guided in the four slit 25c of the base 25. The outer ends of the beams 23b protrude from the slits 25c and the outer surface of the base 25. Further each of the spreaders 23 carry a mandrel 23a perpendicular on the proximal side of the beam 23b. The pin 26 is axially movable in a central guiding formed by the ram 27. The apparatus 20 in figures 5a to 5d is shown in an initial position where the pusher 22 and the ram 27 are each in a proximal position. The beams 23b of the spreader 23 are pressed by support 24 against the distal surface 21b of the shaft 21 and find a radial bearing in the fulcrum 27a for their initial position. Such the four mandrel 23a run parallel to the longitudinal axis L. Further in the initial position of the apparatus 20 the pin 26 is shown in its distal position.

Figures 6a to 6p refer to an exemplary method according to the invention. The method (100) is exemplified on or with the preferred embodiments of cassette unit 10 and apparatus 20 from figures 1a to 5d. Each step (101 to 106) is depicted in a perspective view of a cassette unit and an apparatus in figures 6a,c,e,g,i,k,m,o and a corresponding longitudinal cut in a perspective view of a cassette unit and an apparatus in figures 6b,d,f,h,i,l,n,p.

In figures 6a,b the cassette unit 10 and the apparatus 20 is depicted after a step 101 of initializing the apparatus has taken place. The step 101, 101a is performed by moving the pusher 22 and the ram 27 each in a proximal position whereby each spreader 23 is brought to its first, initial position. For step 101a the pin 26 is additionally moved in its initial distal position.

In figures 6c,d the cassette unit 10 and the apparatus 20 is depicted after a step 102 of inserting the cassette unit has taken place. The step 102 is performed by moving distally the rotationally aligned cassette unit 10 coaxially into the opening 21a of the apparatus 20 whereby the mandrel 23a of each spreader 23 slides into its corresponding bore 14c in the wall 14a of the tube 14 in the cap 12 until the cassette unit finds a limit stop by the proximal surface of the beam 23b of the spreader 23.

In figures 6e,f the cassette unit 10 and the apparatus 20 is depicted after a step 103 of stretching the wall 14a has taken place. The step 103 is performed by moving the pusher 22 axially along the shaft 21 in a distal position to urge each spreader 23 from its initial in a second position whereby the wall 14a with the four bores 14c is circumferentially stretched due to inclination of the four mandrel 23a.

In figures 6g,h the cassette unit 10 with an inserted syringe 30 and the apparatus 20 is depicted after a step 104 of inserting a syringe 30 has taken place. The step 104 is performed by inserting a syringe 30 coaxially by the proximal opening 16 of the cassette unit 10 into the holder 13 until the flange 35 of the syringe 30 has passed the latch 17 on the inner wall of the sleeve 11 whereby the shield 34 is placed within the radially expanded hole 14b defined by the stretched wall 14a. Additionally in a step 104a a syringe 30 may be inserted until the distal end of the shield 34 abuts the proximal end of pin 26 i.e. the limit stop within the hole 14b whereby a proximal section of the pin 26 passes the opening 14e in the cover of the tube 14 or the cap 12.

In figures 6i,j the cassette unit 10 with an inserted syringe 30 and the apparatus 20 is depicted after a step 105 of shrinking the wall has taken place. The step 105 is performed by removing or the ram 27 from its proximal position to free the inner end of the beam 23b of each spreader 23 from the fulcrum 27a, whereby the elastic relaxation of the stretched wall 14a declines the mandrel 23a of each spreader 23 back towards the longitudinal axis L and firmly shrinks at least parts of the wall 14a circumferentially onto at least parts of the outer surface of the shield 34 of the syringe 30. Additionally, the proximally directed bias force of the spring 28 to the support 24 helps to decline the mandrel 23a of each spreader 23 back towards the longitudinal axis L.

In figures 6k,l the cassette unit 10 with an inserted syringe 30 and the apparatus 20 is depicted after a step 106 of removing the cassette unit has partially taken place. The step 106 is performed by removing the cassette unit 10 with the inserted syringe 30 in proximal direction from the apparatus 20 whereby the mandrel 23a of each spreader 23 is retracted from its bore 14c and each spreader 23 is free to move radially and axially in the slit 25c. Additionally in a step 106a the pin 26 is moved in proximal direction synchronously with the distal end of the shield 34 of the syringe 30 while removing the cassette unit 10 until the mandrel 23a of each spreader 23 is retracted from its bore 14c completely.

In figures 6m,n the cassette unit 10 with an inserted syringe 30 and the apparatus 20 is depicted after a step 106 and 106 a of removing the cassette unit has completely taken place with the respective positions of the moveable parts ram 27, pusher 22, spreader 23 and pin 26 after the cassette unit 10 is completely removed from the apparatus 20.

In figures 6o,p the cassette unit 10 with an inserted syringe 30 and the apparatus 20 is depicted after a step 106 and 106 a of removing the cassette unit has completely taken place with the respective positions of the moveable parts ram 27, pusher 22, spreader 23 and pin 26 after the step 101 of initializing the apparatus has again taken place.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. Given the nature of the invention having several aspects contributing to an overall optimum, it is obvious to those skilled in the art that the improvements described, for better clarity, as different aspects, may be applied in any selection and/or combination. For example, a solution provided for an improved assembly of a cap to a prefilled syringe may well be used to improve the assembly of another distinct part e.g. a button or subunit to the drug delivery device. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF REFERENCE NUMERALS

- 10: cassette unit
- 11: sleeve
- 12: cap
- 12a: snap fit
- 13: holder
- 14: tube
- 14a: wall, shroud
- 14b: hole, cavity, interior space, blind hole
- 14c: bore, slit, orifice
- 14d: cover, lid
- 14e: opening, orifice, aperture
- 14f: flange
- 15: window
- 16: opening
- 17: latch

- 20: apparatus
- 21: shaft
- 21a: proximal opening
- 21b: distal surface
- 22: pusher
- 23: spreader
- 23a: mandrel
- 23b: beam
- 24: support
- 25: base
- 25a: trench
- 25b: bushing
- 25c: slit
- 26: pin
- 27: ram
- 27a: fulcrum
- 28: spring
- 30: syringe
- 31: barrel
- 32: needle
- 33: stopper
- 34: shield
- 35: flange
- L: longitudinal axis

- 100: method
- 101: initializing the apparatus
- 101a: initializing the apparatus with pin
- 102: inserting a cassette unit
- 103: stretching the wall
- 104: inserting a syringe
- 104a: inserting a syringe with pin
- 105: shrinking the wall
- 106: removing the cassette unit
- 106a: removing the cassette unit with pin

## Claims

1. A cassette unit (10) for an injection device, the cassette unit (101) comprising:
- a sleeve (11) defining a longitudinal axis (L) with a proximal opening (16)
- a holder (13) integrated coaxially in the sleeve (11) and prepared to accommodate a syringe (30) comprising a barrel (31), a flange (35), a stopper (33), a needle (32) and a shield (34)
- a latch (17) situated near the proximal opening (16) on the inner wall of the sleeve (11) or on the inner wall of the holder (13)
- a cap (12) removably attached to the distal end of the sleeve (11) e.g. by a snap fit (12a)
- a proximally open tube (14) coaxially with the longitudinal axis (L), the tube (14) having a wall (14a) defining a hole (14b) and having a distal cover (14d)
**characterized in that**
the tube (14) is constructed from an elastic material and the wall (14a) forms at least three bores (14c) distributed around the circumference of the wall (14a), each bore (14c) running parallel to the longitudinal axis (L) and piercing the distal cover (14d).

2. The cassette unit (10) for an injection device according to claim 1, wherein the tube (14) is integrated in or forms the distal end of the cap (12).

3. The cassette unit (10) for an injection device according to any of the preceding claims, wherein the wall (14a) of the tube (14) is reinforced around and/or along the bore (14c).

4. The cassette unit (10) for an injection device according to any of the preceding claims, wherein the wall (14a) of the tube (14) is constructed from a highly stretchable material, such as a silicone rubber (LSR), a thermoplastic elastomer (TPE) or a thermoplastic polyurethane (TPU).

5. The cassette unit (10) for an injection device according to any of the preceding claims, wherein the wall (14a) forms four bore (14c) distributed around the circumference of the wall (14a).

6. The cassette unit (10) for an injection device according to any of the preceding claims, wherein the distal cover (14d) forms a flange (14f).

7. The cassette unit (10) for an injection device according to any of the preceding claims, wherein the hole (14b) is adapted to firmly receive at least a part of the shield (34) of the syringe (30) due to the elastic properties of the wall (14a).

8. The cassette unit (10) for an injection device according to any of the preceding claims, wherein in the distal cover (14d) of the tube (14) a central opening (14e) is formed as an orifice or as a slit.

9. An apparatus (20) to assemble a syringe (30) in a cassette unit (10) for an injection device, the apparatus (20) comprising:
- a base (25) having a proximal surface and forming a bushing (25b), the bushing (25b) defining a longitudinal axis (L)
- a support (24) guided coaxially in a trench (25a) formed in the proximal surface of the base (25), the support (24) biased in proximal direction by a spring (28)
- a ram (27) axially guided and moveable in the bushing (25b), the ram (27) forming a circumferential fulcrum (27a) on its proximal end section
- a cylindrical shaft (21) mounted fixedly to the base (25) and proximally spaced above the support (24), the shaft (21) having a proximal opening (21a) to accommodate the distal end of the cassette unit (10) and having a distal surface (21b)
- at least three spreaders (23) distributed around the longitudinal axis (L) between the support (24) and the shaft (21), each spreader (23) having a beam (23b) radially running to the longitudinal axis (L) with its inner end bearable on the fulcrum (27a), the beam (23b) urged by the support (24) towards the distal surface (21b) of the shaft (21) and the outer end of the beam (23b) projecting radially beyond the shaft (21), each spreader (23) having further a mandrel (23a) perpendicularly attached to the inner end of the beam (23b), the mandrel (23a) in a first position of the spreader (23) is running in proximal direction parallel to the longitudinal axis (L)
- a pusher (22) coaxially guided on the shaft (21), the pusher (22) able to move the outer end of the beam (23b) in distal direction
**characterized in that**
due to the distal movement of the pusher (22), the beam (23b) pivots in the fulcrum (27a) and the support (24) is urged distally into the trench (25a) against the force of the spring (28) and the mandrel (23a) is inclined in radial direction with respect to the longitudinal axis (L) in a second position of the spreader (23).

10. The apparatus (20) to assemble a syringe (30) in a cassette unit (10) for an injection device according to claim 9, wherein a movement of the ram (27) in distal direction loosens the inner end of the beam (23b) from the fulcrum (27a) and enables the spreader (23) to move radially inwards.

11. The apparatus (20) to assemble a syringe (30) in a cassette unit (10) for an injection device according to claim 10, wherein a movement of the ram (27) in proximal direction catches the inner end of the beam (23b) into the fulcrum (27a).

12. The apparatus (20) to assemble a syringe (30) in a cassette unit (10) for an injection device according to any of the claims 9 to 11, wherein the surface of the mandrel (23a) is made of or coated with a low-friction compound.

13. The apparatus (20) to assemble a syringe (30) in a cassette unit (10) for an injection device according to any of the claims 9 to 12, the apparatus (20) further comprising a pin (26) running along the longitudinal axis (L) and guided axially in the ram (27), the proximal end of the pin (26) designed to support a distal end of a shield (34) of a syringe (30).

14. A method to assemble a syringe (30) in a cassette unit (10) for an injection device according to claims 1 to 8 carried out by the apparatus (20) according to claims 9 to 13, the method comprising the steps of:
- initializing the apparatus (20) by moving the pusher (22) and the ram (27) each in a proximal position whereby each spreader (23) is brought to its first position
- inserting a cassette unit (10) coaxially by the opening (21a) of the apparatus (20) whereby the mandrel (23a) of each spreader (23) slides into its corresponding bore (14c) in the wall (14a)
- stretching the wall (14a) by moving the pusher (22) in a distal position to urge each spreader (23) in its second position whereby the wall (14a) with the bores (14c) is circumferentially stretched due to the inclined mandrel (23a)
- inserting a syringe (30) coaxially by the proximal opening (16) of the cassette unit (10) into the holder (13) until the flange (35) of the syringe (30) has passed the latch (17, whereby the shield (34) is placed within the radially expanded hole (14b) defined by the stretched wall (14a)
- shrinking the wall (14) by removing the ram (27) from its proximal position to free the inner end of the beam (23b) of each spreader (23) whereby the elastic relaxation of the stretched wall (14b) declines the mandrel (23a) of each spreader (23) back towards the longitudinal axis and firmly shrinks the wall (14a) onto the shield (34) of the syringe (30)
- removing the cassette unit (10) from the apparatus (20) whereby the mandrel (23a) of each spreader (23) is retracted from its bore (14c) and each spreader (23) is free to move radially.

15. The method according to claim 14 to assemble a syringe (30) in a cassette unit (10) for an injection device according to claim 8 carried out on the apparatus (20) according to claim 13, the method further comprising the steps of:
- initializing the apparatus (20) by moving the proximal end of pin (26) into a distal position
- inserting a syringe (30) until the distal end of the shield (34) abuts the proximal end of pin (26) within the hole (14b) whereby the pin (26) passes the opening (16) in the cover (14d) of the tube (14)
- moving the pin (26) in proximal direction synchronously with the distal end of the shield (34) of the syringe (30) while removing the cassette unit (10) until the mandrel (23a) of each spreader (23) is retracted from its bore (14c) completely.
